(19) Europäisches Patentamt

European Patent Office

Office européen des brevets

(11) Publication number: **0 519 950 B1**

## (12) EUROPEAN PATENT SPECIFICATION

(45) Date of publication of patent specification: **01.02.95**     (51) Int. Cl.⁶: **A61K  9/18**

(21) Application number: **91905284.5**

(22) Date of filing: **04.03.91**

(86) International application number:
**PCT/GB91/00324**

(87) International publication number:
**WO 91/13612 (19.09.91 91/22)**

(54) PHARMACEUTICAL COMPOSITION.

| | |
|---|---|
| (30) Priority: **12.03.90 GB 9005498** | (73) Proprietor: **Beecham Group p.l.c.**<br>**SB House**<br>**Great West Road**<br>**Brentford**<br>**Middlesex TW8 9BD (GB)** |
| (43) Date of publication of application:<br>**30.12.92 Bulletin  92/53** | |
| (45) Publication of the grant of the patent:<br>**01.02.95 Bulletin  95/05** | (72) Inventor: **GRATTAN, Timothy, James, Smith-Kline Beecham**<br>**Consumer Brands,**<br>**St. George's Avenue**<br>**Weybridge,**<br>**Surrey KT13 0DE (GB)** |
| (84) Designated Contracting States:<br>**AT BE CH DE DK ES FR GB GR IT LI LU NL SE** | |
| (56) References cited:<br>**EP-A- 0 225 615**<br>**EP-A- 0 294 103**<br>**EP-A- 0 367 746**<br>**US-A- 2 990 332**<br>**US-A- 4 221 778** | (74) Representative: **White, Susan Mary et al**<br>**SmithKline Beecham plc**<br>**Corporate Intellectual Property**<br>**SB House L/5**<br>**Great West Road**<br>**Brentford, Middlesex TW8 9BD (GB)** |

## Description

The present invention relates to controlled, sustained release compositions for pharmaceutical use and in particular to compositions containing an ion-exchange resin having a pharmacologically active drug adsorbed thereon.

Ion-exchange is known to have utility in providing improved drug delivery systems for orally administered drug products.

US Patent 2,900,332 (Keating) discloses drug-resin complexes prepared by interaction of cationic ion-exchange resins with basic drugs in their cationic form, such as amphetamine and codeine. When a drug-resin complex of this type is orally administered, drug release is initiated when the acidic environment of the gastro-intestinal tract is encountered.

Drug-resin complexes thus offered a method for retarding the rate of drug release not available using conventional drug delivery systems. The degree of control of drug release from drug-resin complexes was however found to be generally unsatisfactory since for many drugs only a relatively short delay in drug release was obtained.

Coating of drug-resin particles was therefore undertaken so as to provide a further diffusion barrier to the drug and hence offer greater control over the drug release profile. For example, US Patent 4,221,778 (Raghunathan) discloses a range of drug-resin complexes coated with a water-permeable diffusion barrier coating.

Uncoated complexes formed with the cationic, cross-linked sulphonated polystyrene ion-exchange resins Amberlite XE-69* and Amberlite EX-120*, prepared for the purposes of comparison, are described as having faster dissolution profiles than are desirable for sustained release of drug.

US Patent 4,788,055 (Fischer et al.) describes uncoated complexes prepared from a cross-linked sulphonated polystyrene ion-exchange resin (Amberlite IRP-69) and an acid addition salt of dextromethorphan, conferring a drug-release profile suitable for once in 12 hour dosing, sustained release being achieved by the selection of a drug to resin ratio within the range 1:5 to 1:10.

The above-mentioned publications present results which demonstrate that the rate of release of drug from ion-exchange complexes is influenced by the resinate particle size, with small resinate particles releasing drug more rapidly than large resinate particles.

US 4,788,055 (Fischer et al.) presents results which demonstrate that small resinate particles are considered unsuitable for use in drug-resin complexes where drug release over a time period in excess of 8 hours is desired. A preference is expressed for average particle size in the range 40-80$\mu$m and more preferably in the range 50-70$\mu$m.

Surprisingly, it has now been found that, contrary to the teaching of the prior art, controlled and sustained drug release from drug-resin complexes can be achieved using small resinate particles. Moreover, the use of small resinate particles has been found to confer advantages of practical benefit.

Accordingly, the present invention provides a composition suitable for controlled, sustained release of a pharmaceutically acceptable drug comprising a drug-resin complex formed from an ion-exchange resin and a pharmacologically active drug, characterised in that at least 20% by weight of the resinate particles have a particle size below 35$\mu$m.

Suitably, a composition of the invention has at least 30% by weight and preferably at least 40% by weight of resinate particles having a particle size below 35$\mu$m.

A preferred composition according to the invention is further characterised in that at least 50% by weight and preferably at least 60% by weight of resinate particles have a particle size below 50$\mu$m.

In vivo tests have shown that targeted blood plasma-time profiles may not be achievable from compositions in which resinate particles greater than 50$\mu$m predominate in view of insufficient initial drug release, attributable to the retardation of the rate of release per unit time with increasing particle size.

The present invention overcomes this potential problem. Small resinate particles have been found to confer on a drug-resin complex an increased initial release of drug per unit time which enables therapeutic drug levels to be achieved more rapidly.

Preparations containing suspensions of drug-resin complexes wherein the resinate particle size is greater than about 120$\mu$m are known to impart a gritty texture which renders the product unpalatable for oral consumption, (Irwin et al., Drug Dev. and Ind. Pharm, 13(9-11), 2064, (1987). Panel tests have now shown that particle sizes greater than 50$\mu$m also impart an unpalatable gritty texture to the product. Compositions according to the present invention, in liquid form, also therefore have the advantage over known drug-resin complexes in that they provide a product for oral administration with improved mouth feel.

Furthermore, compositions according to the present invention may confer the additional advantage of enhancing the in vivo bioavailability and/or the duration of action of the drug.

Firstly, release of drug from resinate particles is a diffusion-controlled equilibrium process, and small resin particles offer a more complete release of drug from the resin.

Secondly, the villous structure of the mucosal wall of the gastro-intestinal tract presents a large available surface area. Resinate particles greater than for example 50μm may be too large to enter the interstices between individual villi, such that a large part of the surface area of the mucosal surface may not be available for drug adsorption. It is however possible that small resinate particles may enter between individual villi and hence take a more tortuous path down the gastro-intestinal tract, offering greater availability of the dosage form with the associated potential for a longer duration of action.

In general, all ionisable, orally administrable pharmacologically active drugs that are absorbed under conditions encountered in the gastro-intestinal tract are suitable for use in compositions according to the present invention. Particularly suitable drugs are, for example, those having basic character, including for example phenylpropanolamine, chlorpheniramine, dextromethorphan, noscapine and pseudoephedrine.

Similarly, a wide range of ion-exchange resins is potentially available to form drug-resin complexes. Cationic ion-exchange resins are suitable for use with basic drugs and anionic ion-exchange resins are suitable for use with drug compounds having acidic character. The complex resulting from a cationic ion-exchange resin or an anionic ion-exchange resin will release drug on encountering the ionic environment of the gastro-intestinal tract.

Suitable ion-exchange resins, which it will be appreciated will be pharmaceutically acceptable, are commercially available and include a range of synthetic ion-exchange resins with different polymeric matrices which may be cross-linked.

Ion-exchange resins particularly suitable for use with basic drugs include strongly cationic resins having sulphonic acid groups on a polystryene polymer matrix, which resins are suitably cross-linked, preferably by a divinyl or polyvinyl compound such as divinylbenzene. The proportion of cross-linking may vary from 1 to 20%, suitably from 1 to 12%, for example from 5 to 10%, and preferably from 7 to 9% by weight cross-linking.

Suitable resins are those commercially available under the trade names Amberlite and Duolite* from Rohm and Haas Co. and Dowex* from the Dow Chemical Company. A preferred resin is Amberlite IRP-69.

A basic drug may be presented for complex formation in the form of a pharmaceutically acceptable acid addition salt, in which case the chosen resin will be in its 'sodium' form. Alternatively, the drug may be presented as the free base, in which case the resin will be in its 'hydrogen' form.

Acidic drugs may be presented for complex formation with a suitable anionic ion-exchange resin either as the free acid or in salt form. Suitable salts include inorganic salts for example sodium, potassium, calcium or magnesium salts, or organic salts, for example salts derived from amino acid residues or choline.

It will be appreciated that the invention extends to compositions comprising two or more drugs and two or more ion-exchange resins.

Commercially available ion-exchange resins generally have a particle size distribution in which the average particle size is greater than that required by the present invention, for example Amberlite IRP-69. The size of the ion-exchange particles may therefore be reduced as necessary to achieve the desired particle size distribution. Size reduction may be carried out using standard milling methods. Suitable milling methods include the use of micronisers, commutating mills, pin mills, air-jet mills for example fluid energy mills, and wet milling and ultrasonic techniques.

It will be appreciated that the spread of particle sizes generally present in commercially available ion-exchange resins may allow ion-exchange resin suitable for use in compositions of the present invention to be obtained by sieving. Preferably, ion-exchange resin for use in compositions of the invention is obtained using a combination of size reduction and sieving techniques.

Size reduction and/or sieving may be carried out either before or after loading the ion-exchange resin with drug.

The release profile of drug from a drug-resin complex is influenced by the physico-chemical properties of the drug. Thus the particle size distribution of resinate particles in compositions of the invention may be selected to match the properties of a particular drug, the ion-exchange resin on which it is loaded, and the desired drug-release profile.

Compositions of the invention suitably have a particle size distribution comprising from about 20 to 75% by weight of particles below 35μm and from about 50 to 100% by weight of particles below 50μm, preferably from about 30 to 50% by weight below 35μm and from about 60 to 100% by weight below 50μm.

It will be appreciated that where a drug inherently gives rise to a more rapid release than is desirable for effective sustained release, a proportion (% by weight) of resinate particles of larger size, for example

up to 130μm, may be included to sustain release of drug over the desired time period.

The desired particle size distribution may be achieved by a combination of standard milling and sieving techniques. Where size reduction is carried out before loading the ion-exchange resin with drug, further screening by sieving to achieve a desired composition may also be carried out before drug loading. Alternatively, further screening may be carried out after preparation of the drug-resin complex. This will of course be the procedure adopted where particle size reduction is carried out after loading the drug onto the ion-exchange resin.

Pre-conditioning of ion-exchange resins prior to complex formation may be desirable in order to remove any potentially toxic extractibles and/or to facilitate easier processing and analysis of the resulting resinates.

Adsorption of drug onto ion-exchange resins to form a drug-resin complex may be carried out by standard procedures well known in the art, for example as described in US Patent 2,990,332 (Keating).

Thus, for example, a commercially available ion-exchange resin may be added directly to a solution of drug in an appropriate solvent. The resulting drug-resin complex may then be dried and the resinate particles reduced by milling, and/or screened by sieving into particle size ranges, to achieve the desired particle size distribution.

Alternatively, the drug-resin complex formed initially as described above may be wet-milled and then dried and, as necessary, screened by sieving into particle size ranges.

In a further process for the preparation of drug-resin complexes of the invention, the ion-exchange resin particles are first reduced by milling and/or screened by sieving. The ion-exchange resin having the required particle size distribution is then added to a solution of drug in an appropriate solvent, followed by drying.

Compositions of the invention are suitably administered as liquid suspensions.

The invention further provides a novel process specifically adapted for the preparation of a composition of the invention as a liquid suspension, which process comprises the addition of ion-exchange resin (having the desired particle size distribution wherein at least 20% by weight of the resin particles have a particle size below 35μm) to a mixture of a pharmacologically active drug and a pharmaceutically acceptable liquid carrier.

The mixture of drug and liquid carrier may be in the form of a solution or, where the drug is insoluble in the carrier, in the form of a suspension. It will be appreciated that in situ loading of the drug onto the ion-exchange resin in a liquid carrier is advantageous in that it obviates the need for a separate drying stage.

Compositions according to the present invention will contain drug and ion-exchange resin in a ratio suitable for achieving release of drug over the desired time period. For example, to achieve drug release for up to 12 hours from dosing, it will generally be suitable to use a drug to resin ratio in the range 1:2 to 1:12 by weight, (calculated on a moisture-free basis), the exact ratio being selected to suit the chosen drug.

The release of drug from drug-resin complexes approximates to first-order kinetics. Thus, release rates decrease as the concentration of the drug in the complex decreases. A low drug loading, for example a drug to resin ratio up to 1:30, may therefore be selected to achieve a more prolonged duration of action. However, in order to reduce the cost of materials and the dosage volume of drug-resin complex administered, a drug to resin ratio of 1:2 to 1:10 is generally preferred.

Compositions according to the present invention may be formulated for oral administration in liquid form, for example as a syrup, or alternatively in solid form, for example as a capsule or tablet. Compositions may therefore be formulated in admixture with an appropriate pharmaceutically acceptable vehicle, additionally containing, as desired, pharmaceutically acceptable adjuvants including inter alia thickeners, preservatives, and colouring and flavouring agents.

Accordingly, the present invention provides a pharmaceutical composition comprising a drug-resin complex formed from a pharmaceutically acceptable drug and a pharmaceutically acceptable ion-exchange resin wherein at least 20% by weight of the resinate particles have a particle size below 35μm, in admixture with a pharmaceutically acceptable carrier.

The quantity of resinate in a formulation may be adjusted to deliver an effective dose of a selected drug to a patient in need of treatment. Compositions of the invention are suitably formulated for administration in unit dose form. Thus, the amount of resinate in a composition for oral administration, for example in the form of a capsule or tablet, may be calculated accordingly. As described above, compositions of the invention are particularly suitable for oral administration in liquid form. Liquid compositions may similarly be prepared in unit dose form by presentation for consumption in single-dose liquid-dosage units.

The invention also provides a method of expediting and sustaining therapeutic levels of a pharmaceutically acceptable drug in humans or animals following a single dose, which method comprises administering an effective single dose of a composition comprising a drug-resin complex formed from an ion-exchange resin and a pharmacologically active drug, whereby at least 20% by weight of the resinate particles have a

particle size below 35µm.

In another aspect, the invention provides the use of a composition comprising a drug-resin complex formed from an ion-exchange resin and a pharmacologically active drug for the manufacture of a medicament for use in expediting and sustaining therapeutic drug levels after a single dose, whereby at least 20% by weight of the resinate particles have a particle size below 35µm.

The Examples which follow illustrate the present invention.

## Example 1

### Preparation of small particle ion-exchange resin

Samples of ion-exchange resin having particle sizes below 35µm and 50µm were prepared from Amberlite IRP-69 ion-exchange resin (from Rohm and Haas), a strongly acidic (sodium form), 100-500 wet mesh resin. All samples of Amberlite IRP-69 were conditioned before use according to the following procedure.

Conditioning Procedure: Amberlite IRP-69 was added to an aqueous solution of 2M sodium hydroxide and stirred for one hour. The conditioned resin was isolated by Buchner filtration, washed with successive aliquots of distilled water, and then dried to constant weight at 60°C. [Residual water content = 4.76%].

### Resin Preparation

1(a) Amberlite IRP-69 (100g) was sieved through a 50µ screen. 42g of resin particles below 50µm were collected.

1(b) Resin obtained from Example 1(a) (20g) was sieved through a 35µm screen. 9.2g of resin particles below 35µm were collected.

1(c) Amberlite IRP-69 (2kg) was processed using a fluid energy mill to give 1.6kg of milled material.

1(d) Resin obtained from Example 1(c) (500g) was sieved through a 50µm screen. 455g of resin particles below 50µm were collected.

1(e) Resin obtained from Example 1(d) (20g) was sieved through a 35µm screen. 9.2g of resin particles below 35µm were collected.

1(f) Amberlite IRP-69 (20kg) was processed using a fluid energy mill to give 18kg of milled material.

1(g) Resin obtained from Example 1(f), 10kg, was sieved through a 63µm screen. 9.2kg of resin particles below 63µm were collected.

1(h) Resin obtained from Example 1(g), 20g was sieved through a 35µm screen. 7.5g of resin particles below 35µm were collected.

## Example 2

### Preparation of dextromethorphan-Amberlite IRP-69 resinates

2(a) Dextromethorphan hydrobromide (0.5kg) was dissolved in water (15 litres) at 35°C. Amberlite IRP-69, prepared according to the method of Example 1(d), (2kg; equivalent to 1.9kg of moisture-free resin) was added to the solution. After stirring the mixture for 2 hours, the resinate was isolated by Buchner filtration and washed with successive aliquots of distilled water. The resinate was dried at 50°C under vacuum to give 2.35kg of dextromethorphan-Amberlite IRP-69 resinate particles below 50µm.

2(b) The above procedure was repeated using Amberlite IRP-69 (2kg; equivalent to 1.9kg of moisture-free resin) as received from the supplier. The resulting dried resinate was sieved through a 50µm screen to give 0.9kg of dextromethorphan- Amberlite IRP-69 resinate particles below 50µm. Resinate particles above 50µm were retained for dissolution analysis.

2(c) Dextromethorphan-Amberlite IRP-69 resinate particles below 50µm, prepared according to Example 2(b), were sieved through a 35µm screen.

2(d) Dextromethorphan-Amberlite IRP-69 resinate particles prepared from dextromethorphan HBr (0.5kg) and ion-exchange resin as received from the supplier (2kg; equivalent to 1.9kg of moisture-free resin) were processed using a fluid energy mill to give 0.8kg of milled material. The isolated material was subsequently sieved through a 50µm screen to give 0.74kg of resinate particles below 50µm.

2(e) Dextromethorphan hydrobromide (50g) was dissolved in water (1.5 litres) at 35°C. Amberlite IRP-69, prepared according to the method of Example 1(g), (210g; equivalent to 200g of moisture-free resin) was added to the solution. After stirring the mixture for 1 hour, the resinate was isolated by Buchner filtration and washed with successive aliquots of distilled water. The resinate was dried at 50°C under vacuum to give 230g of dextromethorphan - Amberlite IRP-69 resinate particles below 63μm.

Example 3

Preparation of pharmaceutical syrups containing dextromethorphan-Amberlite IRP-69 resinate

3(a) A suitable syrup base was prepared from sorbitol, keltrol, sodium benzoate, propylene glycol, sodium citrate, citric acid, flavour, colour and water.

3(b) 900ml of syrup base obtained from Example 3(a) was mixed with 19g of the resinate from example 2(d) and sufficient water to give 1 litre of syrup containing the equivalent to 40mg dextromethorphan HBr per 10ml.

3(c) Dextromethorphan hydrobromide (4g) was dissolved in 900ml of the syrup obtained from Example 3(a). The resulting solution was mixed with 16g of resin obtained from Example 1(d) (equivalent to 15.24g of moisture-free resin) and sufficient water to give 1 litre of syrup containing the equivalent to 40mg dextromethorphan HBr per 10ml.

3(d) Dextromethorphan hydrobromide (4g) was dissolved in 900ml of the syrup obtained from Example 3(a). The resulting syrup was mixed with 16.8g of resin obtained from Example 1(g) (equivalent to 16.0g of moisture-free resin) and sufficient water to give 1 litre of syrup containing the equivalent to 40mg dextromethorphan HBr per 10ml.

3(e) Dextromethorphan hydrobromide (3g) was dissolved in 900ml of the syrup obtained from 3(a). The resulting solution was mixed with 12.6g of resin obtained from Example 1(g) (equivalent to 12.0g of moisture-free resin) and sufficient water to give 1 litre of syrup containing the equivalent to 30mg dextromethorphan HBr per 10ml.

3(f) Dextromethorphan hydrobromide (4g) and chlorpheniramine maleate (0.80g) were dissolved in 900ml of the syrup obtained from 3(a). The resulting solution was mixed with 16.0g of resin obtained from Example 1(d) (equivalent to 15.2g of moisture-free resin) and sufficient water to give 1 litre of syrup containing the equivalent to 40mg dextromethorphan HBr per 10ml and 8mg chlorpheniramine maleate per 10ml.

Example 4

In-vitro dissolution profile of dextromethorphan-Amberlite IRP-69 resinate particles

Dextromethorphan-Amberlite IRP-69 resinate particles were prepared as described in Example 2. The in vitro dissolution profile of four samples, each having a drug to resin ratio of 1:3.8 (calculated on a moisture-free basis) and a different particle size distribution, was evaluated by flow-through analysis using a Langenbacher Flow-Through Cell.

Samples of resinate (190mg; equivalent to 40mg of dextromethorphan HBr) were eluted with 0.15M sodium chloride at 4ml/min. The percentage drug release at intervals over an 8 hour time period was determined by UV analysis.

Sample A:     particle size distribution as received from supplier.
Sample B:     particle size >50μm.
Sample C:     particle size <50μm; (at least 20% of particles below 35μm).
Sample D:     particle size <35μm.

| Results | % Drug Release | | | | |
|---|---|---|---|---|---|
| Sample | 1hr | 2hr | 4hr | 6hr | 8hr |
| A | 39 | 58 | 75 | 84 | 91 |
| B | 29 | 47 | 66 | 74 | 78 |
| C | 49 | 69 | 84 | 91 | 96 |
| D | 54 | 76 | 88 | 92 | 97 |

The results show that for Samples A and B, less than 40% of the dose is released within the first hour. In contrast samples C and D, comprising small resinate particles, have released approximately 50% and 55% respectively of their drug load within 1 hour, thus demonstrating the enhanced initial availability of the

EP 0 519 950 B1

drug.

Example 5

Preparation of noscapine-Amberlite IRP-69 resinate
(Drug to resin ratio 1:3.8, calculated on a moisture-free basis using Amberlite IRP-69 with a 4.76% residual moisture content)

5(a) Noscapine HC1 (5g) was dissolved in water (200ml) at 25°C. Amberlite IRP-69 as received from the supplier (20; equivalent to 19g of moisture-free resin) was added to the solution. After stirring for 2 hours the resinate was isolated by Buchner filtration and washed with successive aliquots of distilled water. The resinate was dried at 50°C under vacuum to give 23g of noscapine-Amberlite IRP-69 resinate particles.
5(b) Noscapine-Amberlite IRP-69 resinate particles obtained from Example 5(a) (10g) were sieved through a 50$\mu$m screen. 4.0g of resinate particles below 50$\mu$m were collected.

Example 6

In-Vitro dissolution profile of noscapine-Amberlite IRP-69 resinate

Noscapine-Amberlite IRP-69 resinate particles were prepared as described in Examples 5(a) and 5(b). The in vitro dissolution profile of the two samples, each having a different particle size distribution, was evaluated by flow through analysis using a Langenbacher Flow Through Cell.
Samples of resinate (190mg; equivalent to 40mg noscapine hydrochloride) were eluted using the method described in Example 4.
Sample A:    Particle size distribution as received from supplier
Sample B:    Particle size <50$\mu$m; (at least 20% of particles below 35$\mu$m).

| Results | % Drug Release | | | |
|---------|------|------|------|------|
| Sample | 1hr | 2hr | 4hr | 6hr |
| A | 44 | 63 | 80 | 87 |
| B | 55 | 75 | 87 | 90 |

Example 7

Preparation of phenylpropanolamine-Amberlite IRP-69 resinate
(Drug to resin ratio (1:28.5), calculated on a moisture-free basis using Amberlite IRP-69 with 4.76% residual moisture content)

7(a) Phenylpropanolamine HC1 (3g) was dissolved in water (200ml) at 25°C. Amberlite IRP-69, as received from the supplier, (90g; equivalent to 85.5g of moisture-free resin) was added to the solution. After stirring for 2 hours the resinate was isolated by Buchner filtration and washed with successive aliquots of purified water. The resinate was dried at 50°C under vacuum to give 91g of phenylpropanolamine-Amberlite IRP-69 resinate particles.
7(b) Phenylpropanolamine-Amberlite IRP-69 resinate particles obtained from Example 7(a) (10g) were sieved through a 50$\mu$m screen. 4.0g of resinate particles below 50$\mu$m were collected.
7(c) The procedure described in example 7(a) was repeated using milled and sieved Amberlite IRP-69 resin (Example 1d) instead of the standard grade Amberlite IRP-69. 91g of phenylpropanolamine-Amberlite IRP-69 resinate particles under 50$\mu$m were obtained.

7

Example 8

In-Vitro dissolution profile of phenylpropanolamine-Amberlite IRP-69 resinates

Phenylpropanolamine-Amberlite IRP-69 resinate particles were prepared as described in Example 7.

Samples of resinate (1540mg; equivalent to 50mg phenylpropanolamine hydrochloride) were eluted using the method described in Example 4.

Sample A :     particle size distribution as received from supplier.

Sample B :     particle size > 50µm.

Sample C :     particle size < 50µm; (at least 20% of particles below 35µm).

| Results | % Drug Release | | | |
|---------|------|------|------|------|
| Sample  | 1hr  | 2hr  | 4hr  | 6hr  |
| A       | 44   | 59   | 79   | 88   |
| B       | 29   | 44   | 67   | 76   |
| C       | 47   | 66   | 92   | 97   |

Example 9

Preparation of pseudoephedrine-Amberlite IRP-69 resinate particles
(Drug to resin ratio (1:14.2 calculated on a moisture-free basis using Amberlite IRP-69 with 4.76% residual moisture content)

9(a) Pseudoephedrine hydrochloride (3g) was dissolved in water (200ml) at 25°C. Amberlite IRP-69 as received from supplier (45g; equivalent to 42.8g of moisture-free resin) was added to the solution. After stirring for 2 hours the resinate was isolated by Buchner filtration and washed with successive aliquots of purified water. The resinate was dried at 50°C under vacuum to give 47g of pseudoephedrine-Amberlite IRP-69 resinate particles.

9(b) Pseudoephedrine-Amberlite IRP-69 resinate particles obtained from Example 9(a) (10g) were sieved through a 50µm screem. 4.1g of resinate particles below 50µm were collected.

9(c) The procedure given in Example 9(a) was repeated using milled Amberlite IRP-69 (Example 1d) in place of the standard material. 47g of pseudoephedrine-Amberlite IRP-69 resinate particles below 50µm were collected.

Example 10

In-Vitro dissolution profile of pseudoephedrine-Amberlite IRP-69 resinates.

Pseudoephedrine-Amberlite IRP-69 resinate particles were prepared as described in Example 9.

Samples of resinate (1910mg; equivalent to 120mg psuedoephedrine hydrochloride) were eluted using the method described in Example 4.

Sample A :     particle size distribution as received from supplier.

Sample B :     particle size > 50µ.

Sample C :     particle size < 50µ; (at least 20% particles below 35µm).

| Results | % Drug Release | | | |
|---------|------|------|------|------|
| Sample  | 1hr  | 2hr  | 4hr  | 6hr  |
| A       | 42   | 63   | 83   | 96   |
| B       | 37   | 59   | 82   | 92   |
| C       | 54   | 76   | 91   | 95   |

Example 11

In-Vitro dissolution profile of dextromethorphan-Amberlite IRP-69 resinates particles

Dextromethorphan-Amberlite IRP-69 resinate particles were prepared as described in Example 2(e). The in-vito dissolution profile of two samples, each having a different drug-load, was evaluated by flow-through analysis using a Langenbacher Flow-Through Cell.

The samples of resinate were eluted using the method of Example 4.

Sample A :    148mg; equivalent to 30mg of dextromethorphan HBr.
              Particle size < 63μm (at least 20% of particles below 35μm

Sample B :    197mg; equivalent to 40mg of dextromethorphan HBr.
              Particle size < 63μm (at least 20% of particles below 35μm.

| Results | % Drug Release | | | |
|---------|-----|-----|-----|-----|
| Sample | 1hr | 2hr | 4hr | 6hr |
| A | 48 | 74 | 85 | 93 |
| B | 45 | 64 | 80 | 88 |

Example 12

In-Vitro dissolution profile of dextromethorphanchlorpheniramine-Amberlite IRP-69 resinate particles

A sample of dextromethorphan-Amberlite IRP-69 particles as prepared in Example 2(b) (190mg; equivalent to 40mg of dextromethorphan HBr) with a particle size below 50μm (at least 20% by weight below 35μm) was mixed with chlorpheniramine maleate powder (8mg) and water (5ml). The mixture was allowed to stand for 12 hours, prior to dissolution by the method described in Example 4, in order to effect the loading of the chlorpheniramine onto the resinate.

| Results | % Drug Release | | |
|---------|-----|-----|-----|
| Drug | 1hr | 2hr | 4hr |
| Dextromethorphan HBr | 52 | 67 | 78 |
| Chlorpheniramine maleate | 54 | 68 | 81 |

Example 13

Preparation of dextromethorpahn-Amberlite XE-625 resinate

Amberlite XE-625(250g) was milled using an Apex*commutating mill. The resulting powder was sieved through a 50μm screen. 70g of resin particles below 50μm were collected. The 50μm sieve-cut was shown by further sieve analysis to have 32% by weight of particles below 35μm.

A 10g sample of the 50μm sieve-cut was conditioned with sodium hydroxide solution as described in Example 1. The resulting resin (4g; equivalent to 3.8g moisture-free resin) was treated with dextromethorphan HBr (1g) in water (100ml) according to the method described in Example 2(a).

Example 14

In-Vitro dissolution profile of dextromethorphan-Amberlite XE-625 resinate particles

A sample of the resinate prepared in Example 13 (190mg; equivalent to 40mg of dextromethorphan HBr) was eluted using the method of Example 4.

* The trademarks cited in this application are acknowledged as such.

| Results | % Drug Release | | | |
|---------|------|------|------|------|
| | 1 hr | 2 hr | 4 hr | 6 hr |
| | 42 | 59 | 73 | 82 |

Example 15

Particle size distribution (by weight) of Ion-Exchange Resinates by Sieve Analysis

| Particle Size Range (μm) | Example No. | |
|--------------------------|------|------|
| | 2(a) | 2(e) |
| Passed through 63μm and retained by 40μm. | 42 | 50 |
| Passed through 40μm and retained by 35μm. | 11 | 7 |
| Passed through 35μm. | 47 | 43 |

**Claims**

1. A composition for controlled and sustained release of a pharmaceutically acceptable drug comprising a drug-resin complex formed from an ion-exchange resin and a pharmacologically active drug, characterised in that at least 20% by weight of the resinate particles have a particle size below 35μm.

2. A composition as claimed in claim 1 wherein at least 40% by weight of resinate particles have a particle size below 35μm.

3. A composition as claimed in claim 1 or 2 wherein at least 50% by weight of resinate particles have a particle size below 50μm.

4. A composition as claimed in any one of claims 1 to 3 wherein the pharmacologically active drug has basic character.

5. A composition as claimed in claim 4 wherein the pharmacologically active drug is dextromethorphan, phenylpropanolamine, noscapine, pseudoephedrine or chlorpheniramine, or a pharmaceutically acceptable salt thereof.

6. A composition as claimed in claim 3 or 4 wherein the ion-exchange resin is a strongly cationic resin having sulphonic acid groups on a polystyrene polymer matrix.

7. A composition as claimed in claim 6 wherein the cationic resin is cross-linked with from 1 to 20% by weight of a divinyl or polyvinyl compound.

8. A composition as claimed in claim 7 wherein the cationic resin is cross-linked with from 7-9% by weight of divinylbenzene divinylbenzene.

9. A composition as claimed in claim 8 wherein the ratio of drug to resin is in the range 1:2 to 1:12 by weight, calculated on a moisture-free basis.

10. A composition as claimed in claim 9 comprising a pharmacologically active drug which is dextromethorphan or a pharmaceutically acceptable salt thereof, wherein the ratio of drug to resin is in the range 1:3 to 1:5 by weight, calculated on a moisture-free basis, and the particle size distribution comprises from 30 to 50% by weight of particles below 35μm and from 60 to 100% by weight of particles below 50μm.

**11.** A composition as claimed in any one of claims 1 to 10 further comprising a pharmaceuticlly acceptable carrier.

**12.** A composition as claimed in claim 11 for oral administration in liquid form.

**13.** A process for preparing a composition as defined in any one of claims 1 to 12 comprising the admixture of ion-exchange resin, the pharmacologically active drug and a suitable solvent.

**14.** A process for preparing a composition as defined in claim 12 comprising adding ion-exchange resin as defined in any one of claims 1 to 10 to a mixture of a pharmacologically active drug as defined in any one of claims 1 to 10 and a pharmaceutically acceptable liquid carrier.

**15.** The use of a composition comprising a drug-resin complex formed from an ion-exchange resin and a pharmacologically active drug as defined in any one of claims 1 to 12 for the manufacture of a medicament for use in expediting and sustaining therapeutic drug levels after a single dose.

**Patentansprüche**

**1.** Zusammensetzung zur geregelten Abgabe und Langzeitwirkung eines pharmazeutisch vertraglichen Arzneistoffs, umfassend einen Arzneistoff-Harz-Komplex, gebildet aus einem Ionenaustauscherharz und einem pharmakologisch wirksamen Arzneistoff, dadurch gekennzeichnet, daß mindestens 20 Gew.-% der Harzteilchen eine Teilchengröße unter 35 $\mu$m haben.

**2.** Zusammensetzung nach Anspruch 1, wobei mindestens 40 Gew.-% der Harzteilchen eine Teilchengröße unter 35 $\mu$m haben.

**3.** Zusammensetzung nach Anspruch 1 oder 2, wobei mindestens 50 Gew.-% der Harzteilchen eine Teilchengröße unter 50 $\mu$m haben.

**4.** Zusammensetzung nach einem der Ansprüche 1 bis 3, wobei der pharmakologisch wirksame Arzneistoff basischen Charakter besitzt.

**5.** Zusammensetzung nach Anspruch 4, wobei der pharmakologisch wirksame Arzneistoff Dextromethorphan, Phenylpropanolamin, Noscapin, Pseudoephedrin oder Chlorpheniramin, oder ein pharmazeutisch verträgliches Salz davon ist.

**6.** Zusammensetzung nach Anspruch 3 oder 4, wobei das Ionenaustauscherharz ein stark kationisches Harz ist, mit Sulfonsäuregruppen auf einer Polystyrol-Polymer-Matrix.

**7.** Zusammensetzung nach Anspruch 6, wobei das kationische Harz vernetzt ist mit 1 bis 20 Gew.-% einer Divinyl- oder Polyvinylverbindung.

**8.** Zusammensetzung nach Anspruch 7, wobei das kationische Harz vernetzt ist mit 7 bis 9 Gew.-% Divinylbenzol.

**9.** Zusammensetzung nach Anspruch 8, wobei das Gewichtsverhältnis von Arzneistoff zu Harz im Bereich von 1:2 bis 1:12 liegt, berechnet auf einer feuchtigkeitsfreien Basis.

**10.** Zusammensetzung nach Anspruch 9, umfassend Dextromethorphan oder dessen pharmazeutisch verträgliches Salz als pharmakologisch wirksamen Arzneistoff, wobei das Gewichtsverhältnis von Arzneistoff zu Harz im Bereich von 1:3 bis 1:5 liegt, berechnet auf einer feuchtigkeitsfreien Basis, und die Teilchengrößenverteilung 30 bis 50 Gew.-% Teilchen unterhalb 35 $\mu$m und 60 bis 100 Gew.-% Teilchen unterhalb 50 $\mu$m umfaßt.

**11.** Zusammensetzung nach einem der Ansprüche 1 bis 10, umfassend ferner einen pharmazeutisch verträglichen Träger.

**12.** Zusammensetzung nach Anspruch 11, zur oralen Verabfolgung in flüssiger Form.

EP 0 519 950 B1

**13.** Verfahren zur Herstellung einer Zusammensetzung nach einem der Ansprüche 1 bis 12, umfassend das Vermischen eines Ionenaustauscherharzes, eines pharmakologisch wirksamen Arzneistoffs und eines geeigneten Lösungsmittels.

**14.** Verfahren zur Herstellung einer Zusammensetzung nach Anspruch 12, umfassend die Zugabe eines Ionenaustauscherharzes nach einem der Ansprüche 1 bis 10 zu einem Gemisch eines pharmakologisch wirksamen Arzneistoffs nach einem der Ansprüche 1 bis 10 und einem pharmazeutisch verträglichen flüssigen Träger.

**15.** Verwendung einer Zusammensetzung, umfassend einen Arzneistoff-Harz-Komplex, gebildet aus einem Ionenaustauscherharz und einem pharmakologisch wirksamen Arzneistoff nach einem der Ansprüche 1 bis 12, zur Herstellung eines Arzneimittels zur beschleunigten Einstellung und Aufrechterhaltung therapeutisch wirksamer Arzneistoffspiegel nach einer Einzeldosis.

**Revendications**

**1.** Composition pour la libération contrôlée et retardée d'un médicament acceptable du point de vue pharmaceutique, comprenant un complexe résine-médicament, formé à partir d'une résine échangeuse d'ions et d'un médicament actif du point de vue pharmacologique, caractérisé en ce qu'au moins 20 % m/m des particules de résinate ont une taille de particules inférieure à 35 µm.

**2.** Composition suivant la revendication 1 , dans laquelle au moins 40 % m/m des particules de résinate ont une taille de particules inférieure à 35 µm.

**3.** Composition suivant la revendication 1 ou 2, dans laquelle au moins 50 % m/m des particules de résinate ont une taille de particules inférieure à 50 µm.

**4.** Composition suivant l'une quelconque des revendications 1 à 3, dans laquelle le médicament actif du point de vue pharmacologique a un caractère basique.

**5.** Composition suivant la revendication 4, dans laquelle le médicament actif du point de vue pharmacologique est le dextrométhorphan, la phénylpropanolamine, la noscapine, la pseudoéphédrine, ou la chlorphéniramine, ou un sel de ceux-ci, acceptables du point de vue pharmaceutique.

**6.** Composition suivant la revendication 3 ou 4, dans laquelle la résine échangeuse d'ions est une résine cationique forte comportant des groupes acide sulfonique sur une matrice polymère polystyrène.

**7.** Composition suivant la revendication 6, dans laquelle la résine cationique est réticulée avec 1 à 20 % m/m d'un composé divinylique ou polyvinylique.

**8.** Composition suivant la revendication 7, dans laquelle la résine cationique est réticulée avec 7 à 9 % m/m d'un composé divinylique ou polyvinylique.

**9.** Composition suivant la revendication 8, dans laquelle le ratio médicament/résine est compris entre 1:2 et 1 : 12, calculé en poids sec.

**10.** Composition suivant la revendication 9, comprenant un médicament actif du point de vue pharmacologique, qui est le dextrométhorphan ou un de ses sels, acceptable du point de vue pharmaceutique, dans laquelle le ratio médicament/résine est compris entre 1:3 et 1:5, calculé en poids sec, et la répartition des tailles de particules est comprise entre 30 à 50 % en poids, de particules au dessous de 35 µm, et 60 à 100 % en poids, de particules au dessous de 50 µm.

**11.** Composition suivant l'une quelconque des revendications 1 à 10, comprenant aussi un véhicule acceptable du point de vue pharmaceutique.

**12.** Composition suivant la revendication 11, pour l'administration orale sous forme liquide.

12

**13.** Procédé de préparation d'une composition suivant l'une quelconque des revendications 1 à 12, comprenant le mélange d'une résine échangeuse d'ions, du médicament actif du point de vue pharmacologique, et d'un solvant approprié.

**14.** Procédé de préparation d'une composition suivant la revendication 12, comprenant l'addition d'une résine échangeuse d'ions suivant l'une quelconque des revendications 1 à 10, à un mélange de médicament actif du point de vue pharmacologique suivant l'une quelconque des revendications 1 à 10, et d'un véhicule liquide acceptable du point de vue pharmaceutique.

**15.** Utilisation d'une composition comprenant un complexe médicament/résine formé à partir d'une résine échangeuse d'ions et d'un médicament actif du point de vue pharmacologique suivant l'une quelconque des revendications 1 à 12, pour la fabrication d'une spécialité destinée à être utilisée pour instaurer et maintenir des taux thérapeutiques de médicament après administration d'une dose unique.